(19) Europäisches Patentamt
European Patent Office
Office européen des brevets

(11) Veröffentlichungsnummer: **0 333 659 A2**

# (12) EUROPÄISCHE PATENTANMELDUNG

(21) Anmeldenummer: **89810185.2**

(22) Anmeldetag: **09.03.89**

(51) Int. Cl.4: **C 07 C 157/12**
A 01 N 47/34

(30) Priorität: **18.03.88 CH 1030/88**

(43) Veröffentlichungstag der Anmeldung:
**20.09.89 Patentblatt 89/38**

(84) Benannte Vertragsstaaten:
**AT BE CH DE ES FR GB IT LI NL**

(71) Anmelder: **CIBA-GEIGY AG**
**Klybeckstrasse 141**
**CH-4002 Basel (CH)**

(72) Erfinder: **Farooq, Saleem, Dr.**
**Kirchackerstrasse 27**
**CH-4422 Arisdorf (CH)**

Patentanspruch für folgenden Vertragsstaat:ES

## (54) Substituierte Thioharnstoffderivate.

(57) Neue substituierte N-Thiobenzoyl-N′-alkylthioharnstoffe der Formel

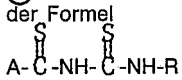 (I),

worin
R $C_1$-$C_5$-Alkyl, $C_3$-$C_6$-Cycloalkyl oder ($C_3$-$C_6$-Cycloalkyl)-$C_1$-$C_3$-Alkyl bedeutet; und A die Bedeutung

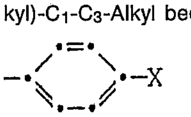 ,

wobei X für Wasserstoff, $C_1$-$C_4$-Alkyl, Trifluormethyl, $C_1$-$C_4$-Alkoxy, $C_1$-$C_4$-Halogenalkoxy mit 1 bis 9 Halogenatomen, Methylthio, Dimethylamino oder Phenyl steht,
oder

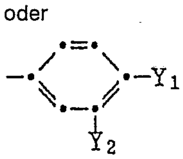 ,

wobei einer der Reste $Y_1$ oder $Y_2$ unabhängig voneinander für Halogen, Methyl oder Phenoxy und die andere für Wasserstoff steht,
oder

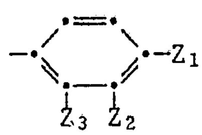 ,

wobei mindestens zwei der Reste $Z_1$, $Z_2$ und $Z_3$ unabhängig voneinander für Halogen oder Methyl stehen und der dritte für Wasserstoff steht,
oder

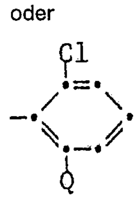 ,

wobei Q Chlor bedeutet oder Wasserstoff mit der Massgabe bedeutet, dass R eine von Methyl abweichende Bedeutung hat; besitzt mit Ausnahme der Verbindungen 1-Methyl-3-thiobenzoyl-thioharnstoff und 1-n-Butyl-3-thiobenzoyl-thioharnstoff. Es

EP 0 333 659 A2

werden beschrieben Verfahren zur Herstellung dieser Verbindungen und ihre Verwendung in der Schädlingsbekämpfung, insbesondere zur Bekämpfung von pflanzenschädigenden Vertretern der Ordnung Akarina.

**Beschreibung**

## Substituierte Thioharnstoffderivate

Die vorliegende Erfindung betrifft neuartige substituierte N-Thiobenzoyl-N′-alkylthioharnstoffe, Verfahren zu ihrer Herstellung und ihre Verwendung in der Schädlingsbekämpfung.

Die erfindungsgemässen Verbindungen haben die Formel I

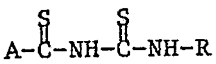

(I),

R $C_1$-$C_5$-Alkyl, $C_3$-$C_6$-Cycloalkyl oder ($C_3$-$C_6$-Cycloalkyl)-$C_1$-$C_3$-Alkyl bedeutet; und A die Bedeutung

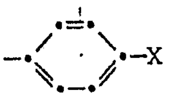

,

wobei X für Wasserstoff, $C_1$-$C_4$-Alkyl, Trifluormethyl, $C_1$-$C_4$-Alkoxy, $C_1$-$C_4$-Halogenalkoxy mit 1 bis 9 Halogenatomen, Methylthio, Dimethylamino oder Phenyl steht,
oder

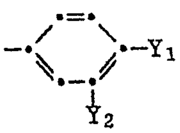

,

wobei einer der Reste $Y_1$ oder $Y_2$ unabhängig voneinander für Halogen, Methyl oder Phenoxy und der andere für Wasserstoff steht,
oder

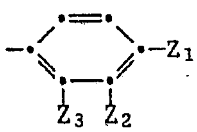

,

wobei mindestens zwei der Reste $Z_1$, $Z_2$ und $Z_3$ unabhängig voneinander für Halogen oder Methyl stehen und der dritte für Wasserstoff steht,
oder

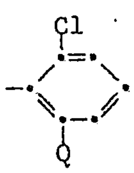

,

wobei Q Chlor bedeutet oder Wasserstoff mit der Massgabe bedeutet, dass R eine von Methyl abweichende Bedeutung hat,
besitzt;
mit Ausnahme der Verbindungen 1-Methyl-3-thiobenzoyl-thioharnstoff und 1-n-Butyl-3-thiobenzoyl-thioharnstoff.

Hervorzuheben sind insbesondere Verbindungen der Formel I, worin R $C_1$-$C_4$-Alkyl oder $C_3$-$C_6$-Cycloalkyl bedeutet; und
A die Bedeutung

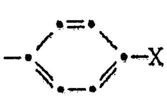

,

wobei X für Wasserstoff, $C_1$-$C_4$-Alkyl, Trifluormethyl, Methoxy, Methylthio oder Dimethylamino steht,
oder

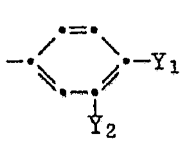

,

wobei einer der Reste $Y_1$ oder $Y_2$ für Halogen und der andere für Wasserstoff steht,
oder

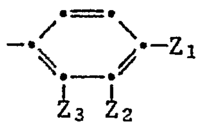 ,

wobei mindestens zwei der Reste $Z_1$, $Z_2$ und $Z_3$ für Halogen stehen und der dritte für Wasserstoff steht,
oder

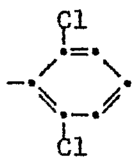

besitzt.

Die Alkylgruppen in Verbindungen der Formel I können geradkettig oder verzweigt sein. Beispiele solcher Gruppen sind u.a. Methyl, Aethyl, Propyl, Isopropyl, n-Butyl and Isomere. Die Cycloalkylgruppen können unsubstituiert oder alkylsubstituiert sein, wie z.B. Cyclopropyl, Methylcyclopropyl, Cyclopentyl usw.

Bei den erfindungsgemässen Verbindungen der Formel I, steht Halogen im wesentlichen für Fluor, Chlor oder Brom.

Insbesondere bevorzugt werden Verbindungen der Formel I, die dadurch gekennzeichnet sind, dass
R $C_1$-$C_4$-Alkyl bedeutet, und
A die Bedeutung

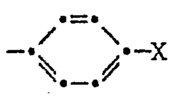 ,

wobei X für Wasserstoff, $C_1$-$C_4$-Alkyl oder Methoxy steht,
oder

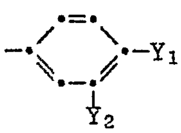 ,

wobei einer der Reste $Y_1$ oder $Y_2$ für Fluor, Chlor oder Brom und der andere für Wasserstoff steht,
oder

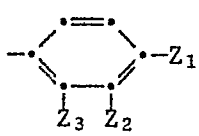 ,

wobei $Z_1$ für Fluor oder Chlor und einer der Rest $Z_2$ und $Z_3$ für Fluor oder Chlor und der andere für Wasserstoff steht,
besitzt.

Weiterhin von Bedeutung sind diejenigen Verbindungen der Formel I, worin A 4-Fluor-, 4-Chlor-, 4-Bromphenyl oder 2,6-Dichlorphenyl bedeutet, sowie diejenigen, worin R Methyl oder Cyclopropyl bedeutet.

Die Verbindungen der Formel I können in an sich bekannter Weise hergestellt werden:
a) Man kann eine Verbindung der Formel I dadurch herstellen (vgl. z.B. Z. Chem. 21, 7, S. 271, 1981; DE-OS Nr. 3337828 und EP-Patentanmeldung Nr. 193249), dass man eine Verbindung der Formel II

S
‖
A-C-$NH_2$ (II)

mit einer Verbindung der Formel III

R-N=C=S (III)

umsetzt, wobei A und R die vorstehend angegebenen Bedeutungen haben.

Das vorerwähnte Verfahren kann üblicherweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff

und Chlorbenzol; Nitrile, wie Acetonitril oder Propionitril; sowie Ketone, z.B. Aceton, Methyläthylketon, Methylisopropylketon und Methylisobutylketon. Das Verfahren wird im allgemeinen bei einer Temperatur von 0 bis 150°C, insbesondere zwischen 10 und 70°C, durchgeführt, vorzugsweise bei Raumtemperatur. Die Durchführung dieses Verfahrens erfolgt vorzugsweise in Gegenwart eines Säureacceptors bzw. einer basischen Substanz. Als Säureacceptoren, bzw. Basen kommen insbesondere tertiäre Amine, wie Trialkylamine und Pyridin, ferner Hydride, Hydroxide, Oxide und Carbonate von Alkali-und Erdalkalimetallen sowie auch Alkalimetallalkoholate, wie z.B. Kalium-t-butylat und Natriummethylat usw., in Betracht.

b) Die Verbindungen der Formel I können auch dadurch hergestellt werden, dass man einen Benzoylthioharnstoff der Formel IV

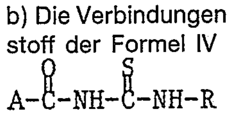 (IV)

mit einer zum Austausch des Carbonylsauerstoffs gegen Schwefel geeigneten Schwefelverbindung behandelt, wobei A und R in Formel IV die vorstehend angegebenen Bedeutungen haben. Als eine solche Schwefelverbindung kommt beispielsweise $P_4S_{10}$ (vgl. z.B. Org. Syn., Coll. Vol., 3, 332, 1955) oder das Lawesson-Reagenz, d.h. das 2,4-Bis(4-methoxyphenyl)-1,3,2,4-dithiadiphosphetan-2,4-dithion (vgl. Kontakte-Merck, 1982, Nr. 3, S. 19; Tetrahedron L. 36, 3047-51, 37, 197-202) in Betracht.

c) Verbindungen der Formel I sind wie nachstehend formelmässig angegeben auch nach der folgenden Arbeitsweise zugänglich (vgl. z.B. J. prakt. Chem., 317, 829, 1975):

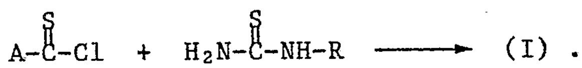

(V) (VI)

In den obigen Formeln V and VI haben A und R die vorstehend angegebenen Bedeutungen.

Die Ausgangsstoffe der Formeln II bis VI sind bekannt oder können, falls sie neu sind, analog bekannten Methoden hergestellt werden. Neue Ausgangspunkte und Zwischenprodukte bilden ebenfalls einen Gegenstand der vorliegenden Erfindung. So können die Thiobenzamide der Formel II wie in J. Chem. 1952, 742 und Anal. pharm. françaises 36 (5-6), 269-72 (1978) angegeben hergestellt werden. Die Benzoylthioharnstoffe der Formel IV können erhalten werden, wie in Chem. Ber. 100 (5), 1602-15 (1967) beschrieben. Die Thiobenzoylchloride der Formel V und die Alkylthioharnstoffe der Formel VI sind herstellbar gemäss Z. Chem. 15 (9), 348 (1975) bzw. Indian J. Chem 4(12), 516-20, (1966).

Die Herstellung von substituierten N-Thioacyl-thioharnstoffen, die im Pflanzenschutz und zur Metallextraktion verwendet werden können, ist aus der DDR-Patentschrift Nr. 214.377 bekannt. Ferner werden in der EP-Patentanmeldung Nr. 193.249 N-Thiobenzoyl-N′-alkylthioharnstoffe als Cytostatika beschrieben. Von den oben genannten Patentveröffentlichungen werden die erfindungsgemäss vorgeschlagenen Thiobenzoylthioharnstoffe der Formel I zwar allgemein umfasst, aber als solche nicht vorbe schrieben. Es wurde überraschenderweise gefunden, dass die erfindungsgemässen, per se neuartigen Verbindungen der Formel I eine ausgeprägte Wirksamkeit als Insektizide, vor allem aber als Akarizide, aufweisen.

Die Verbindungen der Formel I eignen sich zur Bekämpfung von verschiedenartigen Schädlingen an Tieren und Pflanzen sowie im Boden. So können sie zur Bekämpfung von Milben und Zecken der Ordnung Acarina sowie von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera eingesetzt werden.

Die erfindungsgemässen Verbindungen der Formeln I sind vor allem auch wirksam gegen pflanzenschädigende Akariden (Spinnmilben: z.B. der Familien Tetranychidae, Tarsonemidae, Eriophydae, Tyroglyphidae und Glycyphagidae) und daneben auch gegen ektoparasitäre Akariden (Milben und Zecken: z.B. der Familien Ixodidae, Argasidae, Sacroptidae und Dermanyssidae), welche Nutztiere befallen, auf. Einige der erfindungsgemässen Verbindungen zeichnen sich durch gute akarizid-ovizide Aktivität und Blattpenetrationswirkung aus. Die erfindungsgemässen Verbindungen eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsomemus pallidus, Phyllocoptes vitis und Phyllocoptura oleivora.

Verbindungen der Formel eignen sich auch zur Bekämpfung von pflanzenschädigenden Insekten in Zier- und Nutzpflanzen, insbesondere in Baumwollkulturen (z.B. Spodoptera littoralis und Heliothis virescens). Die Verbindungen der Formel I haben auch eine günstige Wirkung gegen Bodeninsekten (z.B. Aulacophora femoralis, Chortophila brassicae, Diabrotica balteata, Pachnoda savignyi und Scotia ypsilon) und gegen saugende Insekten, wie Aphide (z.B. Myzus persicae, Aphis craccivora und Aonidiella aurantii). Ferner sind die erfindungsgemässen Verbindungen wirksam als Ovizide im Pflanzenschutz, speziell zur Bekämpfung von pflanzenschädigenden Insekten, wie z.B. Laspeyresia pomonella und Lobesia botrana.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; pyrethrinartige Verbindungen sowie Karbamate und chlorierte Kohlenwasserstoffe.

Die gute pestizide Wirkung der erfindungsgemässen Verbindungen der Formel I entspricht einer

Abtötungsrate (Mortalität) von mindestens 50-60 % der erwähnten Schädlinge.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnis-sen entsprechend gewählt.

Die Formulierungen, d.h. die mindestens einen Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl-äther, Essigester, Propylmyristat oder Propylpalmitat, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5-Aethylenglykoleinheiten.

Als Beispiele nichtionischer Tenside seien Nonylphenolpolyäthoxyäthanole, Ricinusölpolyglycoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenfalls halogenierte Alkyl-, Benzyl- oder niedrige Hydroxyalkylreste aufweisen. De Salze liegen vorzugsweise als Halogenide, Methylsulfate oder Aethylsulfate vor, z.B. das Stearyltrimethylammoniumchlorid

oder das Benzyldi(2-chloräthyl)-äthylammoniumbromid.

Die in der Formulierungstechnik gebräuchlichen Tenside sind u.a. in folgenden Publikationen beschrieben: "McCutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides, wobei sich die %-Angaben auf das Gewicht beziehen. Während als Handelsware eher konzentrierte Mittel bevorzugt werden, verwendet der Endverbraucher in der Regel verdünnte Mittel bzw. Zubereitungen, die wesentlich geringere Wirkstoffkonzentrationen, wie z.B. 0,1 bis 1000 ppm, aufweisen. Gewöhnlich beträgt die eingesetzte Aufwandmenge der erfindungsgemässen Wirkstoffe der Formel I - insbesondere für landwirtschaftliche Kulturflächen - 0,025 bis 1,0 kg/ha, vorzugsweise 0,1 bis 0,5 kg/ha, beispielsweise 0,1 bis 0,25 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die erfindungsgemässen Verbindungen der Formel I stellen ferner Ausgangsprodukte zur Herstellung von entsprechend substituierten 3-Phenyl-5-imino-1,2,4-dithiozolen dar. Die letztgenannten Verbindungen können durch Oxydation aus den erfindungsgemässen Verbindungen der Formel I hergestellt werden und besitzen wertvolle pestizide und pharmazeutische Eigenschaften.

Beispiel 1: Herstellung von 1-Methyl-3-(4-chlorthiobenzoyl)thioharnstoff

Zu einer Suspension aus 17,2 g 4-Chlorthiobenzamid und 50 ml Acetonitril werden 6,6 g 85%iges Kaliumhydroxyd hinzugefügt. In die nun klare Lösung werden 7,3 g Methylisothiocyanat, gelöst in 10 ml Acetonitril eingetropft. Dabei wird das sich erwärmende Reaktionsgemisch durch Eiskühlung bei einer Temperatur zwischen 20°-25°C gehalten. Danach wird das Reaktionsgemisch noch ca. 15 Minuten bei Raumtemperatur gerührt, und anschliessend werden 100 m Wasser zugegeben. Nach Zugabe von 10 ml konzentriertem HCl wird das ausgefallene gelbe Produkt abfiltriert, mit Wasser gewaschen und aus Aethanol umkristallisiert. Man erhält so die Titelverbindung der Formel

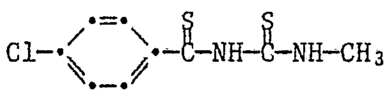

mit einem Schmelzpunkt von 149°-150,5°C (Verbindung Nr. 1).

In entsprechender Weise, wie vorstehend angegeben, werden auch die folgenden Verbindungen der Formel Ia hergestellt:

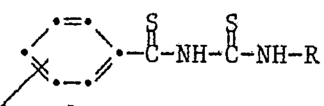

(Ia)

(Phenyl-
substitution)

| Verbindung Nr. | Phenyl-substitution | R | physikalische Daten |
|---|---|---|---|
| 2 | H | $C_2H_5$ | Smp. 68°-70°C |
| 3 | H | $n-C_3H_7$ | Smp. 69°-70°C |
| 4 | H | $i-C_3H_7$ | Smp. 97°-99°C |
| 5 | 3-Cl | $CH_3$ | Smp. 120°-1°C |
| 6 | $2,4-Cl_2$ | $CH_3$ | Smp. 143°-4°C |
| 7 | $2,3-Cl_2$ | $CH_3$ | Smp. 138°-40°C |
| 8 | $3,4-Cl_2$ | $CH_3$ | Smp. 164-6°C |
| 9 | 4-F | $CH_3$ | Smp. 128°-9°C |
| 10 | 4-Br | $CH_3$ | Smp. 159°-60°C |
| 11 | $2,6-Cl_2$ | $CH_3$ | Smp. 223°-4°C |
| 12 | 3-Cl,4-F | $CH_3$ | Smp. 153°-5°C |
| 13 | $4-OCH_3$ | $CH_3$ | Smp. 135°-7°C |
| 14 | H | $n-C_6H_{13}$ | $n_D^{20}=1,6179$ |
| 15 | H | $n-C_{10}H_{21}$ | $n_D^{20}=1,5769$ |
| 16 | $4-t-C_4H_9$ | $CH_3$ | Smp. 109°-11°C |
| 17 | 4-Cl | $i-C_3H_7$ | Smp. 85°-87°C |
| 18 | 3-Cl | $i-C_3H_7$ | Smp. 103°-4°C |
| 19 | $2,4-Cl_2$ | $i-C_3H_7$ | Smp. 135°-6°C |
| 20 | 4-F | $i-C_3H_7$ | Smp. 96°-8°C |
| 21 | $2,3-Cl_2$ | $i-C_3H_7$ | Smp. 171°-2°C |
| 22 | $3,4-Cl_2$ | $i-C_3H_7$ | Smp. 134°-6°C |
| 23 | $2,6-Cl_2$ | $i-C_3H_7$ | Smp. 168°-70°C |
| 24 | 3-Cl,4-F | $i-C_3H_7$ | Smp. 120°-2°C |
| 25 | 4-Br | $i-C_3H_7$ | Smp. 80°-82°C |
| 26 | $4-OCH_3$ | $i-C_3H_7$ | Smp. 88-90°C |
| 27 | $4-t-C_4H_9$ | $i-C_3H_7$ | Smp. 113°-4°C |
| 28 | $4-N(CH_3)_2$ | $CH_3$ | Smp. 149-151°C |

| Verbindung Nr. | Phenyl-substitution | R | physikalische Daten |
|---|---|---|---|
| 29 | 4-Cl | 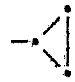 | Smp. 72-74°C |
| 30 | 4-$CF_3$ | $CH_3$ | Smp. 160-162°C |
| 31 | 3,4-$F_2$ | $CH_3$ | Smp. 138-140°C |
| 32 | 4-Cl | n-$C_3H_7$ | Smp. 96-98°C |
| 33 | 4-Cl | n-$C_4H_9$ | Smp. 67-69°C |
| 34 | 4-Cl | $C_2H_5$ | Smp. 88-89°C |
| 35 | 4-F | n-$C_3H_7$ | Smp. 86-87°C |
| 36 | 3,4-$Cl_2$ | n-$C_4H_9$ | Smp. 89-90°C |
| 37 | H | 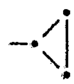 | Smp. 68-69°C |
| 38 | 4-F | 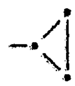 | Smp. 77-79°C |
| 39 | 2,6-$Cl_2$ | 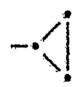 | Smp. 206-208°C |
| 40 | 3,4-$Cl_2$ | 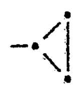 | Smp. 143-145°C |
| 41 | 2-$CH_3$,4-Cl | $CH_3$ | Smp. 156-157°C |
| 42 | 2,4-$(CH_3)_2$ | $CH_3$ | Smp. 104-106°C |
| 43 | 2,4-$(CH_3)_2$ | i-$C_3H_7$ | Smp. 94-95°C |
| 44 | 3,4-$(CH_3)_2$ | 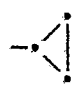 | Smp. 121-122°C |
| 45 | 2,3-$(CH_3)_2$ | $CH_3$ | Smp. 140-142°C |
| 46 | 2,3-$(CH_3)_2$ | i-$C_3H_7$ | Smp. 103-105°C |
| 47 | H | $CH_2$-$C(CH_3)_3$ | Smp. 107-108°C |
| 48 | 4-Cl | $CH_2$-$C(CH_3)_3$ | Smp. 135-137°C |
| 49 | 4-F | $CH_2$-$C(CH_3)_3$ | Smp. 126-128°C |
| 50 | 2,6-$Cl_2$ | $CH_2$-$C(CH_3)_3$ | Smp. 201-202°C |
| 51 | 4-$CF_3$ | $CH_2$-$C(CH_3)_3$ | Smp. 156-157°C |
| 52 | 3,4-$(CH_3)_2$ | $CH_2$-$C(CH_3)_3$ | Smp. 73-74°C |

| Verbindung Nr. | Phenyl-substitution | R | physikalische Daten |
|---|---|---|---|
| 53 | H | 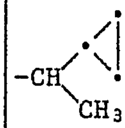 | $n_D^{20} = 1,6665$ |
| 54 | 4-Cl | 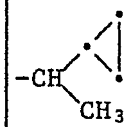 | Smp. 109-111°C |
| 55 | 4-F | 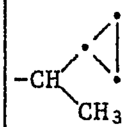 | Smp. 75-77°C |
| 56 | $4-CF_3$ | 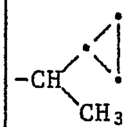 | Smp. 106-108°C |
| 57 | $4-C(CH_3)_3$ | 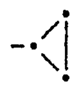 | Smp. 97-98°C |
| 58 | $2,6-Cl_2$ | $C_2H_5$ | Smp. 230-233°C |
| 59 | $2,6-Cl_2$ | $n-C_3H_7$ | Smp. 205-206°C |
| 60 | 2-Cl | 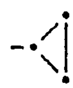 | Smp. 131-132°C |
| 61 | 3-Cl | 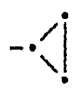 | Smp. 93-95 °C |
| 62 | $4-CF_3$ | 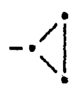 | Smp. 142-144°C |
| 63 | $3,4-F_2$ | 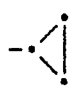 | Smp. 110-111°C |
| 64 | 4-Br | 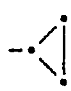 | Smp. 127-129°C |
| 65 | $2-CH_3,4-Cl$ | 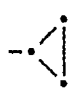 | Smp. 112-113°C |
| 66 | $2,3-Cl_2$ | 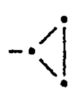 | Smp. 150-152°C |

| Verbindung Nr. | Phenyl-substitution | R | physikalische Daten |
|---|---|---|---|
| 67 | 3-Cl,4-F | 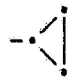 | Smp. 131-133°C |
| 68 | 4-$OCH_3$ | 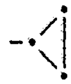 | Smp. 90-92°C |
| 69 | 2-Cl | 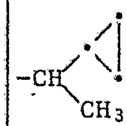 | Smp. 120-121°C |
| 70 | 2,3-$Cl_2$ | 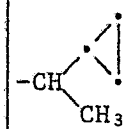 | Smp. 137-139°C |
| 71 | 3-Cl | 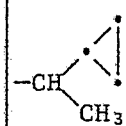 | Smp. 69-71°C |
| 72 | 4-$OCH_3$ | 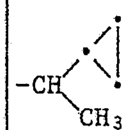 | Smp. 87-89°C |
| 73 | 3,4-$(CH_3)_2$ | 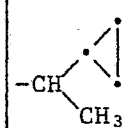 | Smp. 96-98°C |
| 74 | 4-$C(CH_3)_3$ | 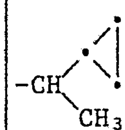 | Smp. 69-71°C |
| 75 | 4- 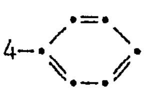 | 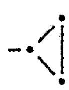 | Smp. 118-120°C |
| 76 | 3-O- 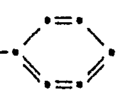 | 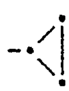 | Smp. 121-123°C |
| 77 | 4-O- 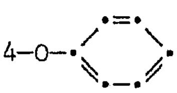 | 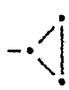 | Smp. 85-86°C |
| 78 | 4-O- 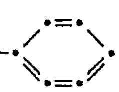 | $CH_3$ | Smp. 135-137°C |

| Verbindung Nr. | Phenyl-substitution | R | physikalische Daten |
|---|---|---|---|
| 79 | 3-O- 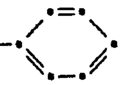 | $CH_3$ | Smp. 108-110°C |
| 80 | 4- 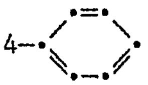 | $CH_3$ | Smp. 175-177°C |

Die folgenden Verbindungen der Formel Ia) sind ebenfalls, wie vorstehend beschrieben, herstellbar:

| Phenyl-substitution | R |
|---|---|
| 4-$SCH_3$ | $CH_3$ |
| 4-$SCH_3$ | i-$C_3H_7$ |
| 4-$N(CH_3)_2$ | i-$C_3H_7$ |
| H | Cyclopentyl |
| H | Cyclohexyl |
| 4-$CF_3$ | i-$C_3H_7$ |

Beispiel 2: Formulierungen für flüssige Wirkstoffe der Formel I, bzw. Ia, gemäss Beispiel 1 (% = Gewichtsprozent)

1. Emulsions-Konzentrate

| | a) | b) | c) |
|---|---|---|---|
| Wirkstoff gemäss Herstellungsbeispielen | 25 % | 40% | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

2. Lösungen

| | a) | b) | c) | d) |
|---|---|---|---|---|
| Wirkstoff gemäss Herstellungsbeispielen | 80 % | 10 % | 5 % | 95 % |
| Aethylenglykol-monomethyläther | 20 % | - | - | - |
| Polyäthylenglykol (MG 400) | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

3. Granulate

| | a) | b) |
|---|---|---|
| Wirkstoff gemäss Herstellungsbeispielen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessnd im Vakuum abgedampft.

4. Stäubemittel

| | a) | b) |
|---|---|---|
| Wirkstoff gemäss Herstellungsbeispielen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungen für feste Wirkstoffe der Formel I, bzw, Ia, gemäss Beispiel 1 (% = Gewichtsprozent)

5. Spritzpulver

| | a) | b) | c) |
|---|---|---|---|
| Wirkstoff gemäss Herstellungsbeispielen | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | a) | b) |
|---|---|---|
| Wirkstoff gemäss Herstellungsbeispielen | 10 % | 10 % |
| Octylphenolpolyäthylenglykoläther (4-5 Mol AeO) | 3 % | - |
| Ca-Dodecylbenzolsulfonat | 3 % | - |
| Ricinusölpolyglykoläther (36 Mol AeO) | 4 % | - |
| Ricinusölthioxilat | - | 25 % |
| Cyclohexanon | 30 % | - |
| Butanol | - | 15 % |
| Xylolgemisch | 50 % | - |
| Essigester | - | 50 % |

Aus diesem Konzentrat können durch Verdünnen mit Wasser Emulsionen jeder gewünschten Konzentration hergestellt werden.

7. Stäubemittel

| | a) | b) |
|---|---|---|
| Wirkstoff gemäss Herstellungsbeispielen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff gemäss Herstellungsbeispielen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff gemäss Herstellungsbeispielen | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff gemäss Herstellungsbeispielen | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Wirkung gegen Tetranychus urticae (OP-sensible) und Tetranychus cinnabarinus (OP-tolerant).

Die Primärblätter von Phaseolus vulgaris-Pflanzen (Buschbohnen) werden mit einem infestierten Blattstück aus einer Massenzucht von Tetranychus urticae (OP-sens.) oder Tetranychus cinnabarinus (OP-tol.) belegt (Mischpopulation). Die Toleranz bezieht sich auf die Verträglichkeit gegenüber Diazinon.

Die so behandelten Pflanzen werden 24 Stunden nach der Infestation mit einer Versuchslösung in Emulsionsform enthaltend 400 ppm der zu prüfenden Verbindung bis zur Tropfnässe besprüht.

Nach 6 (T.urticae) bzw. 7 Tagen (T.cinnabarinus) werden Larven Nymphen, Adulte (alle beweglichen Stadien) sowie abgelegte Eier unter dem Binokular auf lebende und tote Individuen ausgewertet.

Während des Versuchsverlaufs stehen die Pflanzen in Gewächshauskabinen bei etwa 25° C und etwa 50 % rel. Luftfeuchtigkeit, wobei eine Photoperiode um 14 Stunden eingehalten wird.

Verbindungen der Formel Ia) gemäss Beispiel 1 zeigen in diesem Versuch gute Wirkung gegen Tetranychus urticae und Tetranychus cinnabarinus.

Beispiel 4: Ovizide Wirkung auf Tetranychus urticae (OP-resistent)

Eingetopfte Phaseolus vulgaris-Pflanzen (Buschbohnen) im Primärblatt-Stadium (2-Blatt-Stadium) werden pro Blatt jeweils zweimal mit 20 adulten Weibchen von Tetranychus urticae besiedelt. Nach 24-stündiger Eiablage werden die Weibchen mit einer Saugpumpe (Wasserstrahlpumpe) von den Pflanzen entfernt, so dass auf diesen nur noch die abgelegten Milbeneier (d.h. 50 bis 100 Eier pro Test-Pflanze) verbleiben.

Die mit den Milbeneiern infestierten Pflanzen werden dann mit einer wässrigen Emulsion enthaltend 400 ppm der zu prüfenden Verbindung biz zur Tropfnässe besprüht und 6 Tage bei 25° C und etwa 50 % relativer Luft feuchtigkeit gehalten, wobei eine Photoperiode von 14 Stunden eingehalten wird. Nach dieser Zeit wird die prozentuale Mortalität der Eier und eventuell geschlüpfter Larven durch Auszählen bestimmt.

Verbindungen der Formel Ia) gemäss dem vorstehenden Beispiel 1 zeigen gute Wirkung in obigem Test.

Beispiel 5: Pflanzenmitizide Blattpenetrations-Wirkung auf Tetranychus cinnabarinus

Für den Versuch werden eingetopfte Baumwollpflanzen im 4-Blatt-Stadium, die mit Tetranychus cinnabarinus infestiert sind, verwendet. Die Besiedlung mit den Milben erfolgt 1 Tag vor der Wirkstoffapplikation.

Die Blattoberseiten der mit diesen Milben infestierten Versuchspflanzen werden mit einer wässrigen Emulsionszubereitung enthaltend 400 ppm des zu prüfenden Wirkstoffes besprüht. Nach dem Antrocknen des Sprühbelages wird jeweils der Rand der Blattoberseite von einer Anzahl befallener Blätter mit einem Wulst aus zähflüssigem Leim (Raupenleim) abgegrenzt, um ein Ueberlaufen der Milben von der Blattunterseite auf die Blattoberseite zu verhindern.

Die behandelten Pflanzen werden dann im Gewächshaus bei einer Temperatur von etwa 22° C (Nacht) bis 25° C (Tag) und einer relativen Luftfeuchtigkeit von ca. 50 % gehalten mit einer Photoperiode von 14 Stunden. Sieben Tage nach der Wirkstoffapplikation wird festgestellt, ob eine translaminare Wirkung, d.h. Blattpenetration des Wirkstoffs von der Blattoberseite zur Blattunterseite, eingetreten war, und zwar durch Bestimmung der Mortalität der Eier, Larven, Nymphen sowie adulten Stadien auf den unbehandelten Blattunterseiten.

Verbindungen der Formel Ia) gemäss dem vorstehenden Beispiel 1 zeigen gute Wirkung in obigem Test.

Beispiel 6: Wirkung gegen Panonychus ulmi (OP und Carb. resistent)

Eingetopfte Apfelsämlinge mit etwa 20-30 gut ausgebildeten Blättern werden mit jeweils 3 adulten Weibchen von Panonychus ulmi pro Blatt besiedelt. Nach sieben Tagen werden die infestierten Pflanzen mit einer wässrigen Emulsion enthaltend 400 ppm der zu prüfenden Verbindung bis zur Tropfnässe besprüht. Die behandelten Pflanzen werden dann während weiterer 14 Tage bei ca. 25°C und etwa 50 % relativer Luftfeuchtigkeit mit einer Photoperiode von 14 Stunden im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen der Formel Ia) gemäss dem vorstehenden Beispiel 1 zeigen gute Wirkung im obigen Test.

Beispiel 7: Wirkung gegen tierparasitäre Milben

Von mit Dermanyssus gallinae befallenen Hühnern werden Ansätze bestehend aus etwa 50 Milben verschiedener Stadien (Larven, Nymphen und Imagines) entnommen. Die Milbenansätze werden jeweils mit einer wässrigen Emulsion, Suspension bzw. Lösung enthaltend 800 ppm des zu prüfenden Wirkstoffes benetzt. Hierzu werden die Milbenansätze in einem Teströhrchen mit der den Wirkstoff enthaltenden flüssigen Zubereitung übergossen; die Flüssigkeit wird anschliessend mit Hilfe eines Wattebausches aufgesogen. Die so behandelten Milben verbleiben während 72 Stunden in dem Teströhrchen. Nach dieser Zeit wird die prozentuale Abtötung der behandelten Milben gegenüber unbehandelten Kontrollansätzen ermittelt.

Verbindungen der Formel Ia gemäss dem vorstehenden Beispiel 1 zeigen gute Wirkung im obigen Test.

Beispiel 8: Wirkung gegen Zecken

Als Testtiere werden mit Blut vollgesogene adulte Weichen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten dorsal fixiert und dann mit einem mit einer wässrigen Emulsion bzw. Lösung enthaltend 400 ppm der zu untersuchenden Verbindung getränkten Wattebausch während 1 Stunde bedeckt. Nach Entfernung des Wattebausches werden die Zecken über Nach bei 24°C getrocknet und anschliessend in einem klimatisierten Raum bei konstanten Bedingungen (28°C, 80 % rel. Luftfeuchtigkeit) gehalten, und zwar für 4 Wochen bis zum Ende der Eiablage und Beginn des Larvenschlupfes. Zur Auswertung wird die Mortalität, die prozentuale Hemmung der Ablage von fertilen Eiern (Blockierung der Embryogenese bzw. des Schlupfes) gegenüber unbehandelten Kontrollen ermittelt.

Die Verbindungen der Formel Ia) gemäss Beispiel 1 zeigen gute Wirkung in obigem Test.

Beispiel 9: Wirkung gegen Zecken: Abtötungswirkung in verschiedenen Entwicklungsstadien

Als Testobjekte werden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die Testtiere werden für kurze Zeit in wässrige Emulsionen der zu untersuchenden Substanzen mit einer Konzentration von 800 ppm getaucht. Die in Teströhrchen befindlichen Emulsionen werden dann mit Watte aufgenommen und die benetzten Testtiere in den so kontaminierten Röhrchen belassen. Die Auswertung (% - Mortalität) erfolgt für Larven nach 3 Tagen und für Nymphen und Imagines nach 14 Tagen.

Verbindungen der Formel Ia) gemäss den Beispiel 1 sind in diesem Test gut wirksam.

Beispiel 10: Kontaktwirkung auf Aphis craccivora

In Töpfen angezogene 4-5 Tage alte Erbsenkeimlinge werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 mg der zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22°C und einer rel. Luftfeuchtigkeit von etwa 55 % durchgeführt.

Die Verbindungen der Formel 1a) gemäss Beispiel 1 zeigen gute Wirkung in diesem Test.

Beispiel 11: Frassgift-Wirkung auf Laodelphax striatellus und Nilaparvata lugens (Nymphen);

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer wässerig/acetonischen Lösung (1:1) enthaltend 400 ppm des Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage on der behandelten Pflanze gehalten. Die Auswertung auf % Mortalität erfolgt 1, 4 und 8 Tage nach der Behandlung.

Die Verbindungen der Formel Ia) gemäss Beispiel 1 zeigen gute Wirkung in diesem Test.

Beispiel 12: Insektizide Frassgift-Wirkung gegen Spodoptera littoralis

Ca. 25 cm hohe eingetopfte Baumwollpflanzen werden mit wässrigen Wirkstoffemulsionen besprüht, die den Wirkstoff in einer Konzentration von 400 ppm enthalten.

Nach dem Antrocknen des Sprühbelages werden die Baumwollpflanzen mit Spodoptera littoralis-Larven im ersten larvalen Stadium besiedelt. Der Versuch wird bei 24°C und etwa 60 % relativer Luftfeuchtigkeit durchgeführt. Nach 120 Stunden wird die prozentuale Mortalität der Test-Insekten gegenüber unbehandelten Kontrollen bestimmt.

Die Verbindungen der Formel Ia) gemäss Beispiel 1 zeigen gute Wirkung in diesem Test.

Beispiel 13: Ovizide Wirkung auf Heliothis virescens

Entsprechende Mengenanteile einer benetzbaren pulverförmigen Formulierrung, enthaltend 25 Gew. % des zu prüfenden Wirkstoffes, werden mit so viel Wasser vermischt, dass sich eine wässrige Emulsion mit einer Wirkstoffkonzentration von 400 ppm ergibt.

In diese wirkstoffhaltige Emulsion werden eintägige Eigelege von Heliothis auf Cellophan® während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt.

Die Verbindungen der Formel Ia) gemäss Beispiel 1 zeigen im obigen Test gute Wirkung.

Beispiel 14: Systemische Wirkung auf Nilaparvata lugens (Wasser)

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 400 ppm enthält

und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wird dann mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N 2 bis N 3 Stadium besiedelt und mit einem Plastikzylinder abgedeckt. Der Versuch wird bei 20°C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff die Testtiere an den oberen Pflanzenteilen abtötet.

Die Verbindungen der Formel 1a) gemäss Beispiel 1 zeigen im obigem Test gute Wirkung.

Beispiel 15: Wirkung gegen Bodeninsekten (Diabrotica balteata)

Es werden 5 Maiskeimlinge von 1-3 cm Länge sowie eine Filterpapier-Rondelle in eine wässrige Wirkstofflösung enthaltend etwa 4 Vol.-% Aceton eingetaucht. Der Wirkstoffgehalt der verwendeten Lösung beträgt 400 ppm. Die eingetauchte Filterpapier-Rondelle wird auf den Boden eines Kunststoff-Bechers (Inhalt 200 ml) gelegt und darauf wird eine trockene Filterpapier-Rondelle sowie die Maiskeimlinge und 10 Larven von Diabrotica balteata im 2. oder 3. Larvalstadium gegeben. Der Ansatz wird bei ca. 24°C und 40-60 % relativer Luftfeuchtigkeit und Tageslicht gehalten. Die Bonitur erfolgt nach 6 Tagen gegenüber unbehandelten Kontrollansätzen.

Die Verbindungen der Formel Ia) gemäss Beispiel 1 zeigen in diesem Test gute Wirkung.

**Patentansprüche**

1. Verbindungen der Formel I

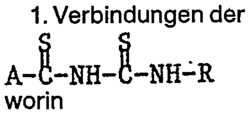 (I),

worin

R $C_1$-$C_5$-Alkyl, $C_3$-$C_6$-Cycloalkyl oder ($C_3$-$C_6$-Cycloalkyl)-$C_1$-$C_3$-Alkyl bedeutet; und

A die Bedeutung

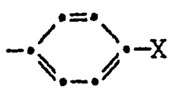 ,

wobei X für Wasserstoff, $C_1$-$C_4$-Alkyl, Trifluormethyl, $C_1$-$C_4$-Alkoxy, $C_1$-$C_4$-Halogenalkoxy mit 1 bis 9 Halogenatomen, Methylthio, Dimethylamino oder Phenyl steht,

oder

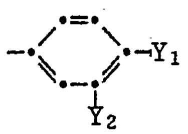 ,

wobei einer der Reste $Y_1$ oder $Y_2$ unabhängig voneinander für Halogen, Methyl oder Phenoxy und der andere für Wasserstoff steht,

oder

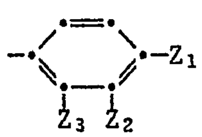 ,

wobei mindestens zwei der Reste $Z_1$, $Z_2$ und $Z_3$ unabhängig voneinander für Halogen oder Methyl stehen und der dritte für Wasserstoff steht,

oder

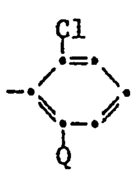 ,

wobei Q Chlor bedeutet oder Wasserstoff mit der Massgabe bedeutet, dass R eine von Methyl abweichende Bedeutung hat,

besitzt;

mit Ausnahme der Verbindungen 1-Methyl-3-thiobenzoyl-thioharnstoff und 1-n-Butyl-3-thiobenzoyl-thioharnstoff.

2. Verbindung der Formel I gemäss Anspruch 1, dadurch gekennzeichnet, dass
R $C_1$-$C_4$-Alkyl oder $C_3$-$C_6$-Cycloalkyl bedeutet; und
A die Bedeutung

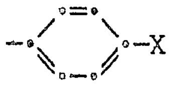 ,

wobei X für Wasserstoff, $C_1$-$C_4$-Alkyl, Trifluormethyl, Methoxy, Methylthio oder Dimethylamino steht, oder

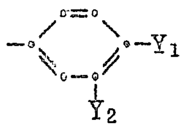 ,

wobei einer der Reste $Y_1$ oder $Y_2$ für Halogen und der andere für Wasserstoff steht, oder

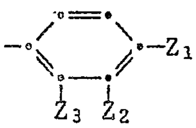 ,

wobei mindestens zwei der Reste $Z_1$, $Z_2$ und $Z_3$ für Halogen stehen und der Dritte für Wasserstoff steht, oder

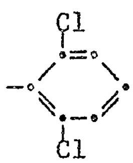

besitzt.

3. Verbindungen der Formel I gemäss Anspruch 1 oder 2, dadurch gekennzeichnet, dass Halogen Fluor, Chlor oder Brom bedeutet.

4. Verbindung der Formel I, gemäss einem der Ansprüche 1 bis 3, dadurch gekennzeichnet, dass
R $C_1$-$C_4$-Alkyl bedeutet; und
A die Bedeutung

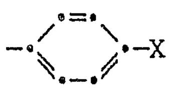 ,

wobei X für Wasserstoff, $C_1$-$C_4$-Alkyl oder Methoxy steht, oder

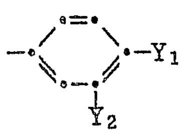 ,

wobei einer der Reste $Y_1$ oder $Y_2$ für Fluor, Chlor oder Brom und der andere für Wasserstoff steht, oder

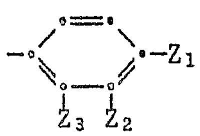 ,

wobei $Z_1$ für Fluor oder Chlor und einer der Reste $Z_2$ und $Z_3$ für Fluor oder Chlor und der andere für Wasserstoff steht,
besitzt.

5. Verbindungen der Formel I gemäss Anspruch 4, dadurch gekennzeichnet, dass A 4-Fluorphenyl, 4-Chlorphenyl, 4-Bromphenyl oder 2,6-Dichlorphenyl bedeutet.

6. Verbindungen der Formel I gemäss einem der Ansprüche 1 bis 5, dadurch gekennzeichnet, dass R Methyl bedeutet.

7. Verbindungen der Formel I gemäss einem der Ansprüche 1 bis 5, dadurch gekennzeichnet, dass R Cyclopropyl bedeutet.

8. Verbindung gemäss Anspruch 5 der Formel

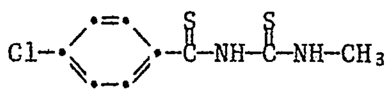 .

9. Verbindung gemäss Anspruch 5 der Formel

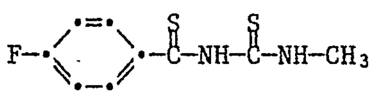 .

10. Verbindung gemäss Anspruch 5 der Formel

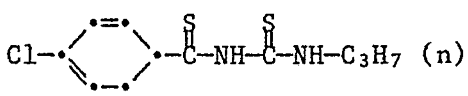 .

11. Verbindung gemäss Anspruch 6 der Formel

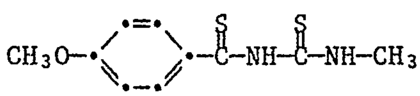 .

12. Verbindung gemäss Anspruch 6 der Formel

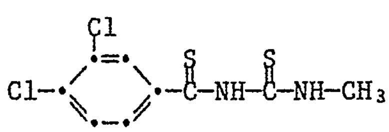 .

13. Verbindung gemäss Anspruch 7 der Formel

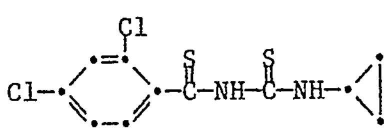

14. Verbindung gemäss Anspruch 7 der Formel

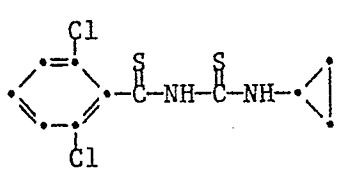 .

15. Verbindung gemäss Anspruch 6 der Formel

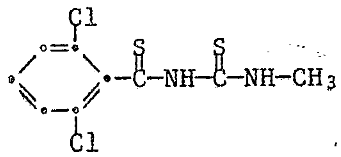

16. Verbindung gemäss Anspruch 6 der Formel

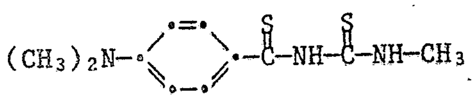

17. Verfahren zur Herstellung einer Verbindung der Formel I gemäss Anspruch 1, dadurch gekennzeichnet, dass man eine Verbindung der Formel II

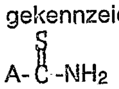 (II)

mit einer Verbindung der Formel III

R-N=C=S (III)

umsetzt, wobei A und R die unter Anspruch 1 angegebenen Bedeutungen haben.

18. Verfahren zur Herstellung einer Verbindung der Formel I gemäss Anspruch 1, dadurch gekennzeichnet, dass man eine Verbindung der Formel IV

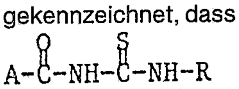 (IV)

mit einer zum Austausch des Carbonylsauerstoffs gegen Schwefel geeigneten Schwefelverbindung behandelt, wobei A und R die unter Anspruch 1 angegebenen Bedeutungen haben.

19. Schädlingsbekämpfungsmittel, welches als aktive Komponente eine Verbindung gemäss einem der Ansprüche 1 bis 16 zusammen mit geeigneten Trägern und/oder anderen Zuschlagstoffen enthält.

20. Verwendung einer Verbindung gemäss einem der Ansprüche 1 bis 16 zur Bekämpfung von Insekten und Vertretern der Ordnung Akarina an Tieren und Pflanzen.

21. Verwendung gemäss Anspruch 20 zur Bekämpfung von pflanzenschädigenden Insekten und Vertretern der Ordnung Akarina.

22. Verwendung nach Anspruch 21 zur Bekämpfung pflanzenschädigender Spinnmilben.

23. Verfahren zur Bekämpfung von Insekten und Vertretern der Ordnung Akarina, dadurch gekennzeichnet, dass man die Schädlinge bzw. deren verschiedene Entwicklungsstadien oder ihren Aufenthaltsort mit einer pestizid wirksamen Menge einer Verbindung der Formel I gemäss einem der Ansprüche 1 bis 16 oder mit einem Mittel enthaltend neben Zusatz- und Trägerstoffen eine pestizid wirksame Menge dieser Verbindung, in Kontakt bringt oder behandelt.

**Patentansprüche für folgenden Vertragsstaat:ES**

1. Verfahren zur Herstellung einer Verbindung der Formel I

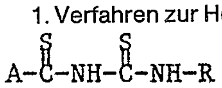 (I),

worin

R $C_1$-$C_5$-Alkyl, $C_3$-$C_6$-Cycloalkyl oder ($C_3$-$C_6$-Cycloalkyl)-$C_1$-$C_3$-Alkyl bedeutet; und

A die Bedeutung

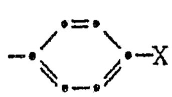 ,

wobei X für Wasserstoff, $C_1$-$C_4$-Alkyl, Trifluormethyl, $C_1$-$C_4$-Alkoxy, $C_1$-$C_4$-Halogenalkoxy mit 1 bis 9 Halogenatomen, Methylthio, Dimethylamino oder Phenyl steht,

oder

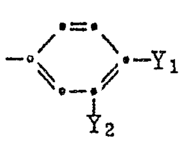 ,

wobei einer der Reste $Y_1$ oder $Y_2$ unabhängig voneinander für Halogen, Methyl oder Phenoxy und der andere für Wasserstoff steht,

oder

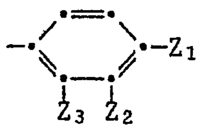 ,

wobei mindestens zwei der Reste $Z_1$, $Z_2$ und $Z_3$ unabhängig voneinander für Halogen oder Methyl stehen und der dritte für Wasserstoff steht,
oder

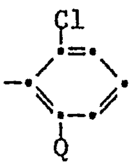 ,

wobei Q Chlor bedeutet oder Wasserstoff mit der Massgabe bedeutet, dass R eine von Methyl abweichende Bedeutung hat,
besitzt;
mit Ausnahme der Verbindungen 1-Methyl-3-thiobenzoyl-thioharnstoff und 1-n-Butyl-3-thiobenzoyl-thioharnstoff, dadurch gekennzeichnet, dass man
a) eine Verbindung der Formel II

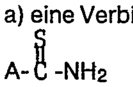 (II)

mit einer Verbindung der Formel III
R-N=C=S (III)
umsetzt; oder
b) eine Verbindung der Formel IV

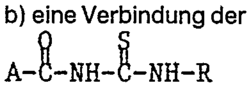 (IV)

mit einer zum Austausch des Carbonylsauerstoffs gegen Schwefel geeigneten Schwefelverbindung behandelt;
wobei in den Formeln II, III und IV A und R die vorstehend angegebenen Bedeutungen haben.

2. Verfahren gemäss Anspruch 1 zur Herstellung einer Verbindung der Formel I, worin
R $C_1$-$C_4$-Alkyl oder $C_3$-$C_6$-Cycloalkyl bedeutet; und
A die Bedeutung

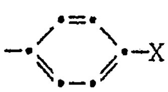 ,

wobei X für Wasserstoff, $C_1$-$C_4$-Alkyl, Trifluormethyl, Methoxy, Methylthio oder Dimethylamino steht,
oder

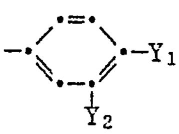 ,

wobei einer der Reste $Y_1$ oder $Y_2$ für Halogen und der andere für Wasserstoff steht,
oder

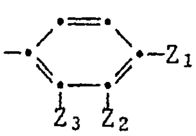 ,

wobei mindestens zwei der Reste $Z_1$, $Z_2$ und $Z_3$ für Halogen stehen und der dritte für Wasserstoff steht,
oder

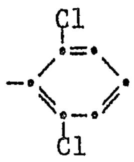

besitzt.

3. Verfahren gemäss Anspruch 1 oder 2 zur Herstellung einer Verbindung der Formel I, worin Halogen Fluor, Chlor oder Brom bedeutet.

4. Verfahren gemäss einem der Ansprüche 1 bis 3 zur Herstellung einer Verbindung der Formel I, worin
R $C_1$-$C_4$-Alkyl bedeutet; und
A die Bedeutung

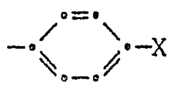 ,

wobei X für Wasserstoff, $C_1$-$C_4$-Alkyl oder Methoxy steht,
oder

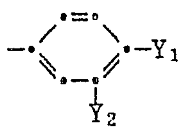 ,

wobei einer der Reste $Y_1$ oder $Y_2$ für Fluor, Chlor oder Brom und der andere für Wasserstoff steht,
oder

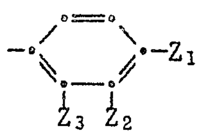 ,

wobei $Z_1$ für Fluor oder Chlor und einer der Reste $Z_2$ und $Z_3$ für Fluor oder Chlor und der andere für Wasserstoff steht,
besitzt.

5. Verfahren gemäss Anspruch 4 zur Herstellung einer Verbindung der Formel I, worin
A 4-Fluorphenyl, 4-Chlorphenyl, 4-Bromphenyl oder 2,6-Dichlorphenyl bedeutet.

6. Verfahren gemäss einem der Ansprüche 1 bis 5 zur Herstellung einer Verbindung der Formel I, worin
R Methyl bedeutet.

7. Verfahren gemäss einem der Ansprüche 1 bis 5 zur Herstellung einer Verbindung der Formel I, worin
R Cyclopropyl bedeutet.

8. Verfahren gemäss Anspruch 5 zur Herstellung der Verbindung

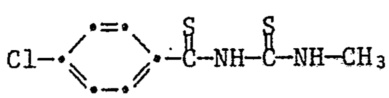 .

9. Verfahren gemäss Anspruch 5 zur Herstellung der Verbindung

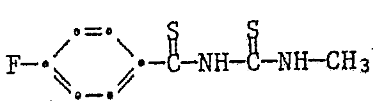 .

10. Verfahren gemäss Anspruch 5 zur Herstellung der Verbindung

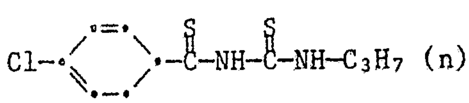 .

11. Verfahren gemäss Anspruch 6 zur Herstellung der Verbindung

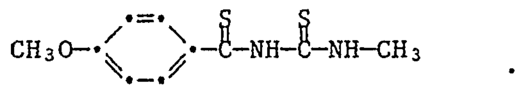

12. Verfahren gemäss Anspruch 6 zur Herstellung der Verbindung

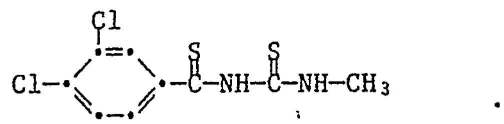

13. Verfahren gemäss Anspruch 7 zur Herstellung der Verbindung

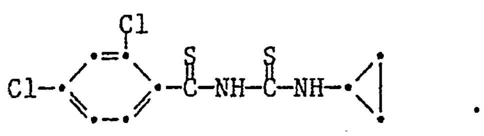

14. Verfahren gemäss Anspruch 7 zur Herstellung der Verbindung

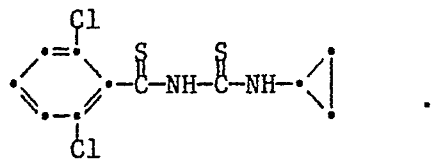

15. Verfahren gemäss Anspruch 6 zur Herstellung der Verbindung

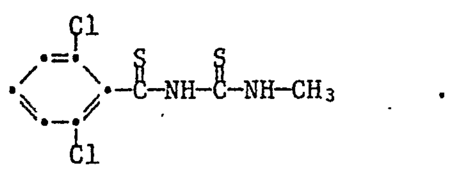

16. Verfahren gemäss Anspruch 6 zur Herstellung der Verbindung